# NOUVEAU FASCICULE DE BREVET EUROPEEN

(11) **EP 1 062 936 B2**
(45) Date de publication et mention de la décision concernant l'opposition: **25.02.2009**
(45) Mention de la délivrance du brevet: 18.12.2002
(21) Numéro de dépôt: 00401512.9
(22) Date de dépôt: 29.05.2000
(51) Int. Cl.: A61K 8/18

(54) **Composition de cosmétique capillaire sous forme d'émulsion d'eau-dans-silicone comprenant au moins un polymère fixant**
Kosmetische Zusammensetzung für das Haar, die in Form einer Wasser-in-Silicon-Emulsion vorliegt und ein fixierendes Polymer enthält
Hair cosmetic composition as a water-in-silicone emulsion containing at least a fixing polymer

(30) Priorité: 18.06.1999 FR 9907769
(43) Date de publication de la demande: 27.12.2000
(73) Titulaire: L'ORÉAL, 75008 Paris (FR)
(72) Inventeur: Pasquet, Dorothée, 92270 Bois-Colombes (FR); Belli, Emmanuelle, 75018 Paris (FR)
(74) Mandataire: Dossmann, Gérard

(56) Documents cités:
- EP-A- 0 407 089
- EP-A- 0 435 483
- EP-A- 0 490 582
- EP-A- 0 715 842
- EP-A- 0 761 214
- EP-A- 0 847 748
- EP-A- 0 914 814
- WO-A1-85/03641
- DE-A- 4 229 922
- DE-A- 19 642 622
- JP-A- 04 370 152
- WENNINGER J.A. ET AL: 'International Cosmetic Ingredient Dictionary and Handbook', vol. 2, partie 7TH ED 1997, THE COSMETIC, TOILETRY, AND FRANGRANCE ASSOCIATION, WASHINGTON, DC pages 1087, 88 - 1649, 50

## Description

L'invention a pour objet une composition cosmétique capillaire, se présentant sous forme d'émulsion eau-dans-silicone et comprenant au moins un polymère fixant. Elle vise également son utilisation pour la fabrication de formulations capillaires telles que des produits de soin, des produits de conditionnement ou des produits pour la fixation et/ou le maintien des cheveux.

Les compositions cosmétiques capillaires contiennent, en général, au moins un polymère de préférence anionique, non ionique ou amphotère. Ils peuvent, par exemple, apporter des propriétés de fixation des cheveux.

Les formulations les plus couramment utilisées se présentent le plus souvent sous forme de mousse aérosol, de spray aérosol, de spray flacon pompe ou bien de gel.

Les mousses permettent généralement d'obtenir sur les cheveux une bonne répartition des compositions cosmétiques et elles sont, en outre, d'une utilisation aisée. Les polymères utilisés dans ces produits étant généralement non moussants ou faiblement moussants, il est nécessaire d'ajouter un agent moussant et/ou un agent améliorant la qualité de la mousse.

Les agents moussants et/ou les agents améliorant la qualité de la mousse habituellement utilisés sont par exemple des agents tensioactifs anioniques, non ioniques ou amphotères. Toutefois, ces agents tensioactifs provoquent parfois un remoussage sur cheveux mouillés, ce qui nuit à une réalisation rapide et soignée de la coiffure.

Par ailleurs, l'application de mousse sur les cheveux présente parfois l'inconvénient d'alourdir la coiffure. Enfin, la réalisation de mousses nécessite obligatoirement un dispositif aérosol.

Les sprays aérosols permettent une répartition rapide et homogène du produit sur la chevelure, et donnent par exemple de très bonnes qualités de maintien et de tenue de la coiffure. Cependant, ils présentent quelquefois l'inconvénient de donner un toucher collant à l'application, et rêche après séchage: leurs qualités cosmétiques sont limitées. Par ailleurs, ils nécessitent, tout comme les mousses, l'utilisation d'un dispositif aérosol.

Parmi les documents de l'art antérieur décrivant des compositions cosmétiques capillaires se présentant notamment sous la forme de mousses ou de sprays, le document EP 260 641 décrit des émulsions comprenant 0,1 à 5 % en poids d'un copolymère diméthylpolysiloxane/polyoxyalkylène ayant un point trouble compris entre 20 et 40 °C ; 0,05 à 10 % d'une huile de silicone et 0,1 à 10 % d'un polymère fixant soluble dans l'eau ou un mélange d'eau et d'alcool ainsi que des agents tensioactifs ioniques ou non ioniques, dans un mélange hydroalcoolique.

Les gels sont une autre forme galénique couramment utilisée pour apporter, par exemple, un maintien efficace à la coiffure, grâce aux polymères qu'ils contiennent. On observe toutefois certains inconvénients comme une texture alourdissante à l'application - due à la présence de certains épaississants, un toucher collant, une coiffure peu naturelle et un manque de douceur.

De manière surprenante et inattendue, la Demanderesse a découvert, contre toute attente, qu'il était possible de réaliser des compositions cosmétiques capillaires qui ne présentent pas les inconvénients exprimés ci-dessus, en choisissant, en tant que support cosmétique, une émulsion particulière d'eau et de silicone, qui sera définie plus en détail par la suite.

L'invention a pour objet une composition cosmétique capillaire se présentant sous la forme d'émulsion eau-dans-silicone, caractérisée en ce qu'elle comprend, dans un milieu cosmétiquement acceptable:
(I) entre 10 % et 30 % en poids d'une silicone non oxyalkylénée choisie(s) parmi les silicones linéaires, cycliques, volatiles ou non, arylées ou non;
(II) au moins une silicone oxyalkylénée émulsionnante dans une proportion de 0.1 à 10 % en poids, et
(III) au moins un polymère fixant anionique, cationique, amphotère ou non ionique.
ladite silicone oxyalkylénée étant choisie parmi les composés de formule générale (I): 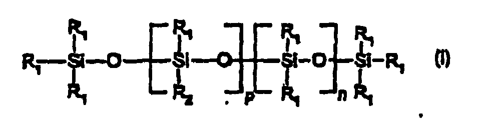 formule dans laquelle :
- R₁, identique ou différent, représente un atome d'hydrogène, un radical alkyle linéaire ou ramifié en C₁-C₃₀ ou un radical phényle,
- R₂, identique ou différent, représente -(CₓH₂ₓ)-(OC₂H₄)ₐ-(OC₃H₆)_{b}-OR₃,
- R₃, identique ou différent, est choisi parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 0 à 1000,
- p varie de 1 à 8,
- a varie de 0 à 50,
- b varie de 0 à 50,
- a + b est supérieur ou égal à 1,
- x varie de 1 à 5,
le poids moléculaire moyen en nombre étant supérieur ou égal à 15.000 et de préférence compris entre 25.000 et 75.000, ayant une viscosité mesurée au moyen du Rhéomat RM 180 à 25°C, avec une lecture après 30 secondes supérieure à 0,5 Pas à un taux de cisaillement de 200 s⁻¹.

Un autre objet de l'invention concerne un procédé cosmétique capillaire mettant en oeuvre cette composition.

Un autre objet de l'invention concerne plus particulièrement un procédé de fixation et/ou de maintien des cheveux mettant en oeuvre cette composition.

Encore un autre objet de l'invention concerne l'utilisation de cette composition pour la fabrication de produits capillaires, notamment ceux destinés à la fixation et/ou la mise en forme de la coiffure.

Au sens de la présente invention, on entend par émulsion eau-dans-silicone, une dispersion dont la phase discontinue est formée par une phase aqueuse et dont la phase continue est formée par une phase grasse, la phase continue étant principalement constituée de ladite silicone.

Les compositions cosmétiques capillaires conformes à la présente invention présentent avantageusement une viscosité mesurée au moyen du Rhéomat RM 180, à 25 °C, avec une lecture après 30 secondes supérieure à 1 Pas, à un taux de cisaillement de 200 s⁻¹.

Au sens de la présente invention, on entend par silicone non oxyalkylénée, tout polymère ou oligomère organosilicié à structure linéaire ou cyclique, ramifiée ou réticulée, de poids moléculaire variable, obtenu par polymérisation et/ou polycondensation de silanes convenablement fonctionnalisés, et constitué pour l'essentiel par une répétition de motifs principaux dans lesquels les atomes de silicium sont reliés entre eux par des atomes d'oxygène en formant une liaison siloxane ≡Si-O-Si≡, des radicaux hydrocarbonés, éventuellement substitués, étant directement liés d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

Les silicones non oxyalkylénée peuvent être modifiées, par exemple par des groupements carboxyliques.

On peut, par exemple, utiliser les silicone non oxyalkylénée à fonction carboxylique répondant à la formule II': 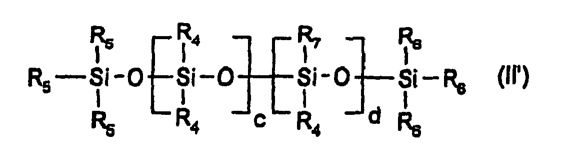 dans laquelle:
- les radicaux R₄ sont identiques ou différents et sont choisis dans le groupe comprenant les radicaux alkyles en C₁-C₂₂, linéaires ou ramifiés, les radicaux alcoxy en C₁-C₂₂ et les radicaux phénylés,
- les radicaux R₅, R₆, R₇ sont identiques ou différents et sont choisis dans le groupe comprenant les radicaux alkyles en C₁-C₂₂, linéaires ou ramifiés ou les radicaux alcoxy en C₁-C₂₂,
- c et d sont des nombres allant de 0 à 1000, la somme c + d allant de préférence de 2 à 1000.

On utilise avantageusement les produits commercialisés sous la marque Huile M 642, SLM 23 000/1 ou SLM 23 000/2 par la Société WACKER, ou encore sous la marque 176-12057 par la Société GENERAL ELECTRIC, ou encore sous la marque FZ 3703 par la Société OSI, ou encore sous la marque BY 16 880 par la Société TORAY SILICONE.

De préférence, la masse moléculaire en nombre du polymère siliconé est comprise entre 10 000 et 1 000 000 environ, et encore plus préférentiellement entre 10 000 et 100 000 environ.

D'autres silicones non oxyalkylénées convenant particulièrement bien pour la mise en oeuvre de la présente invention sont les silicones comprenant au moins un substituant contenant au moins deux groupements, identiques ou différents, choisis parmi les acides carboxyliques ou leurs sels, les amides et les esters, l'un au moins de ces groupements étant un acide carboxylique ou ses sels.

Ces silicones comprennent de préférence au moins un motif de formule IV':

ZRₐSiO_{(3-a)/2} (IVʹ)

dans laquelle formule IV' Z est un radical répondant à la formule V' suivante: 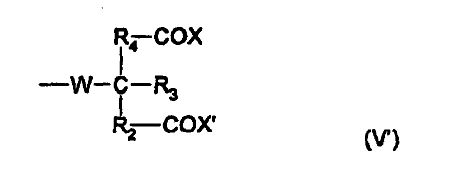 dans laquelle formule V' :
- W, R₂ et R₄, identiques ou différents, sont choisis parmi une liaison covalente et un radical alkylène linéaire ou ramifié ayant de 1 à 6 atomes de carbone pouvant comprendre un groupement hydroxyle,
- R₃ désigne un atome d'hydrogène, un radical alkyle linéaire ou ramifié en C₁-C₆,
   X et X', identiques ou différents, sont choisis parmi les radicaux OM, NR₅R₆ et OR₇,
- M désigne un atome d'hydrogène, un métal alcalin (par exemple Na⁺, K⁺), NH₄,⁺ les groupements ammonium comportant un reste choisi dans le groupe comprenant les aminoacides basiques tels que la lysine, l'arginine, la sarcosine, l'ornithine, la citrulline et les aminoalcools tels que la monoéthanolamine, la diéthanolamine, la triéthanolamine, la glucamine, la N-méthyl glucamine, l'amino-3 propanediol-1,2,
- R₅ et R₆, identiques ou différents, sont choisis dans le groupe comprenant l'hydrogène et les alkyles linéaires ou ramifiés en C₁- C₆ ou bien R₅ et R₆ peuvent former ensemble un hétérocycle à 5 ou 6 chaînons tel que la morpholine,
- R₇ est choisi parmi les radicaux alkyles linéaires ou ramifiés en C₁-C₃₀,
- l'un au moins des groupements X et X' désigne OM,

Dans la formule IV, les radicaux R, identiques ou différents, sont choisis parmi les radicaux alkyles notamment en C₁-C₁₀ et en particulier méthyle, les radicaux fluoroalkyles notamment en C₁-C₁₀, les radicaux aryles en C₆-C₁₂ et en particulier phényle; a est choisi parmi 0, 1 et 2 et de préférence 1 ou 2.

Les autres unités de la silicone sont, de préférence, choisies parmi celles de formule VI':

R_{b}SiO_{(4-b)/2} (VI')

dans laquelle R a la même signification que pour la formule IV et b est égal à 0, 1, 2 ou 3 et de préférence égal à 2 ou 3.

Les silicones comprenant au moins une unité de formule IV' sont notamment décrites dans le brevet US 4 931 062. De telles silicones sont par exemple commercialisées sous la marque SLM 23 105 par la société WACKER et sous la marque DENSODRIN OF par la société BASF.

Par silicone volatile, on entend selon la présente invention, toute silicone présentant une pression de vapeur mesurable et en particulier qui mesurée à 25°C à la pression atmosphérique (10⁵ Pa) est de préférence supérieure à 0,01 mm Hg (2,6 Pa). On emploie de préférence des huiles dont le point d'ébullition à la pression atmosphérique est de l'ordre de 80 à 260°C.

Parmi les silicones volatiles, on peut citer:
- (i) les silicones volatiles cycliques ayant de 3 à 7 atomes de silicium et de préférence de 4 à 5 pouvant répondre à la formule suivante (VIII) : 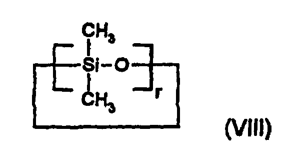
dans laquelle r varie de 3 à 7 (bornes incluses).

Il s'agit par exemple de la cyclotétradiméthylsiloxane, de la cyclopentadiméthylsiloxane ou de la cyclohexadiméthylsiloxane,
- (ii) les silicones volatiles linéaires ayant de 2 à 9 atomes de silicium. Elles peuvent répondre à la formule suivante (IX) : 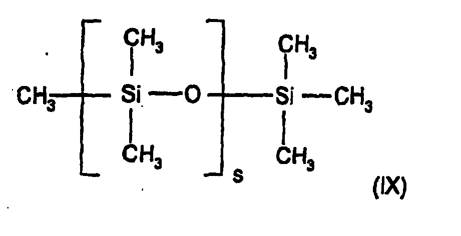
dans laquelle s varie de 1 à 8 (bornes incluses).
Il s'agit par exemple de l'hexaméthyldisiloxane ou de l'octaméthyltrisiloxane. Les silicones volatiles sont préférentiellement la cyclotétradiméthylsiloxane et l'hexaméthyldisiloxane.

Par silicone non volatile, on entend selon la présente invention, toute silicone présentant une pression de vapeur mesurée à 25°C à la pression atmosphérique (10⁵ Pa) de préférence inférieure à 0,01 mm Hg (2,6 Pa).

Les silicones non volatiles peuvent être modifiées ou non. Parmi les silicones non modifiées, on peut utiliser par exemple les huiles, les gommes ou les résines de silicone.

Les silicones arylées non volatiles comportent au moins un radical de type aryle éventuellement substitué. Les radicaux aryles sont par exemple le phényle, naphtyle, benzyle ou phénéthyle.

Les silicones arylées non volatiles présentent préférentiellement la formule suivante (X): 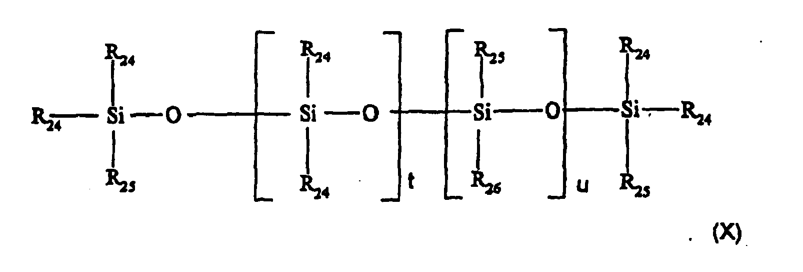 dans laquelle :
R₂₄, identique ou différent, désigne un radical alkyle en C₁-C₁₀.
R₂₆, identique ou différent, désigne un groupement aryle, ce groupement aryle pouvant comprendre un ou plusieurs cycles aryle, éventuellement substitués.
R₂₅, identique ou différent, désigne R₂₆, R₂₄ ou Si(R₂₄)₃
t varie de 0 à 1000,
u varie de 1 à 1000,
la somme t+u peut varier de 1 à 2000.

Les substituants des groupements aryles peuvent être des groupes alkyles, alkényles, acyles, cétones, halogènes (par exemple Cl et Br), amines. Des exemples de groupements aryles sont phényle, un groupement phényle substitué par des radicaux alkyle ou des radicaux alkényle en C₁-C₅ tel que allylphényle, méthylphényle, éthylphényle, vinylphényle et leurs mélanges.

De préférence , R₂₄ désigne un radical méthyle. De préférence , R₂₆ désigne un radical phényle. De préférence , R₂₅ désigne un radical méthyle, phényle ou triméthylsilyle. Plus particulièrement, la somme t+u varie de 1 à 1000.

Parmi les composés de formules (X), on peut utiliser par exemple la phényl triméthicone, la diphényl diméthicone ou la phényl diméthicone (dénominations INCI 5ème édition 1993).

A titre d'exemple de ces composés, on peut citer ceux vendus par la société BAYER sous le nom Huile BAYSILONE FLUID PD5, par la société DOW CORNING sous le nom DOW CORNING 556 FLUID, par RHONE POULENC sous les noms MIRASIL DPDM, RHODORSIL HUILE 510V100, RHODORSIL HUILE 550, RHODORSIL HUILE 510V500, RHODORSIL HUILE 710, sous les dénomination WACKER BELSIL PDM 20, PDM 200, PDM 1000 par la société WACKER.

En tant que silicone arylée, on peut utiliser également une silicone comprenant au moins un groupement aryle, comme les huiles de polyphénylméthylsiloxane ou de polydiméthyldiphénylsiloxane.

Les silicones arylées particulièrement visées par la présente invention sont celles décrites dans le brevet français FR 2 745 173 appartenant à la Demanderesse déposé le 22/02/1996.

Les silicones arylées utilisées conformément à l'invention sont notamment les huiles de polyalkylarylsiloxane du type de celles décrites dans l'encyclopédie Kirk-Othmer, 4ème ed., vol. 22.

Comme autre type de silicone modifiée non oxyalkylénée, on peut citer celles modifiées par des groupements alcoxylés, ou sulfonates, ou thiols, ou hydroxylés, ou aminés, ou hydroxyacylamino. De préférence, on choisira la silicone non oxyalkylénée parmi les silicones volatiles ou les silicones arylées non volatiles ou les silicones à groupements carboxyliques.

Les huiles de polyalkylarylsiloxane préférées sont les huiles de polydiméthyldiphénylsiloxane et les huiles de polyphénylméthylsiloxane.

On peut citer, à titre d'exemple, les huiles de polydiméthyldiphénylsiloxane vendues par la Société Rhône-Poulenc sous la marque Silbione et les huiles de polyphénylméthylsiloxane commercialisées par la Société Goldschmidt sous la marque ABIL AV20-200-350-1000. On peut également citer la silicone phénylée commercialisée par Dow Corning sous la marque DC 556.

De façon préférentielle, on utilise les silicones oxyalkylénées de formule générale (I) qui répondent à au moins une des, et de préférence à toutes les, conditions suivantes :
- R₁ désigne le radical méthyle.
- R₃ représente un atome d'hydrogène, un radical méthyle ou un radical acétyle et de préférence hydrogène.
- p varie de 2 à 6.
- a est compris entre 5 et 40 et de préférence entre 15 et 30.
- b est compris entre 5 et 40 et de préférence entre 15 et 30.
- x est égal à 2 ou 3.
- n varie de 20 à 600, de préférence de 50 à 500 et encore plus particulèrement de 200 à 500.

De telles silicones sont par exemple décrites dans le brevet US 4 311 695 qui est indu à titre de référence.

Les silicones les plus particulièrement préférées sont par exemple celles vendues en solution à 10% en poids dans une cyclométhicone (DOW CORNING 344) sous la dénomination commerciale FLUID DC 3225 C par la société DOW CORNING ou celle vendue sous la dénomination SILWET L 7001 par la société UNION CARBIDE.

Les polymères fixants (B) cationiques, anioniques, amphotères et non ioniques utilisables conformément à l'invention sont décrits ci-après.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 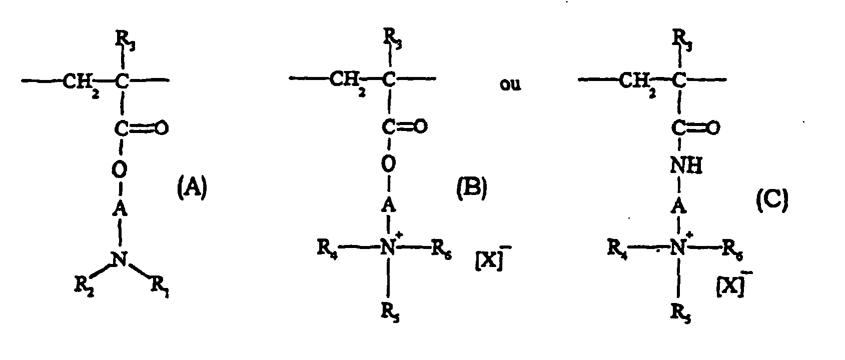 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - et le copolymère vinylpyrrolidone / méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que les gommes de guar contenant des groupements cationiques trialkylammonium.
   De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole tels que les produits commercialisés par BASF sous l'appellation Luviquat TFC;
(4) les chitosanes ou leurs sels;
   les sels utilisables sont en particulier les acétate, lactate, glutamate, gluconate ou le pyrrolidone carboxylate de chitosane.
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont un poids moléculaire compris entre environ 500 et 5.000.000.
1) Les groupements carboxyliques sont apportés par des monomères mono ou diacides carboxyliques insaturés tels que ceux répondant à la formule: 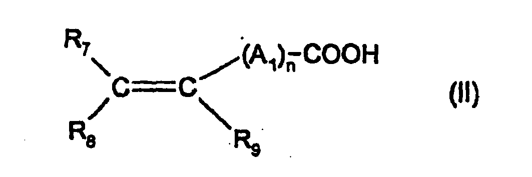 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
   Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α-ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinylétherlanhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinylelacide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle /néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;
B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quatemisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butyl aminoéthyle, de N, N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de polyaminoamides de formule générale III: 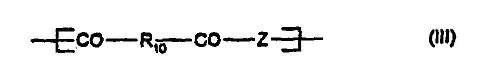 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical IV 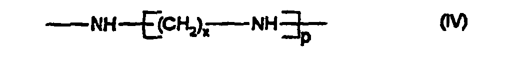 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 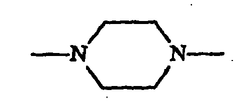
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule V: 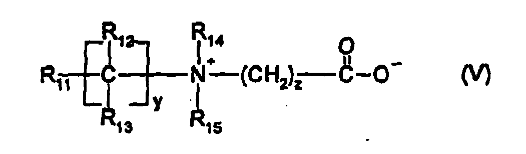 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle /diméthyl carboxyméthylammonio éthylméthacrylate de méthyle tel que le produit vendu sous la dénomination DIAFORMER Z301 par la société SANDOZ.
(5) les polymères dérivés du chitosane comportant des motifs monomères répondant aux formules suivantes : 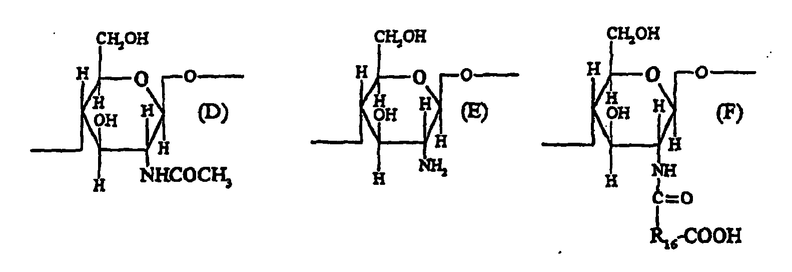 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 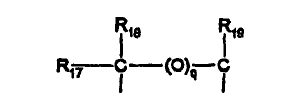 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) par exemple décrits dans le brevet français 1 400 366 : 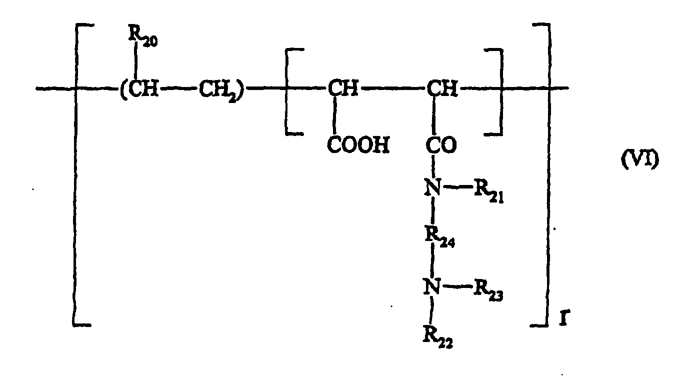 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus, ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (VII)

      où D désigne un radical 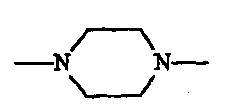 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII')
   où D désigne un radical 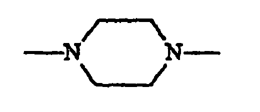 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées. Les gommes de guar non ioniques non modifiées sont par exemple les produits vendus sous la dénomination VIDOGUM GH 175 par la société UNIPECTINE et sous la dénomination JAGUAR C par la société MEYHALL.
Les gommes de guar non-ioniques modifiées utilisables selon l'invention sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

Les polymère non ionique qui conviennent tout particulièrement pour la réalisation des compositions conformes à l'invention sont ceux choisis parmi:
* les copolymères de vinyllactame tels que les copolymères de vinylpyrrolidone et d'acétate de vinyle et les copolymères vinylpyrrolidone/ acétate de vinyle/ propionate de vinyle
* la polyvinylcaprolactame LUVISKOL PLUS (BASF)
* les homopolymères d'acétate de vinyle tels que APPRETAN EM (HOECHST) ou RHODOPAS A 012 (RHONE POULENC)
* les polyalkyloxazolines tels que PEOX 50 000 et PEOX 500 000 (DOW CHEMICAL)
* les copolymères d'acétate de vinyle et d'ester acrylique tels que le RHODOPAS AD 310 (RHONE POULENC)
* les copolymères d'acétate de vinyle et d'éthylène tels que APPRETAN TV (HOECHST)
* les copolymères d'acétate de vinyle et d'ester maléique tels que APPRETAN MB EXTRA (HOECHST)
* les homopolymères d'acrylates d'alkyle et les homopolymères de métacrylates d'alkyle tels que LUHYDRAN A 848 S (BASF)
* les copolymères d'esters acryliques tels que PRIMAL AC-261 K (ROHM & HAAS), ACRONAL 601 (BASF) ou APPRETAN N 9213 (HOECHST)
* les copolymères d'acrylonitrile et d'un monomère non-ionique tels que CJ 0601 B (ROHM & HAAS)
* les polyuréthanes tels que ACRYSOL RM 1020 ou ACRYSOL RM 2020 (ROHM & HAAS)
* les copolymères d'acrylate d'alkyle et d'uréthane tels que 8538-33 (NATIONAL STARCH)
* les polyamides tels que ESTAPOR LO 11 (RHONE POULENC)

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 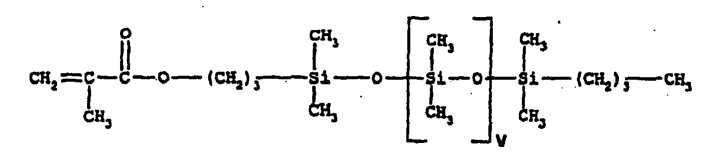 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères fixants, des polyuréthannes fonctionnalisés ou non, siliconés ou non.

Les polyurétannes particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

La silicone de non oxyalkylénée est présente dans la composition à une concentration relative en poids préférentiellement comprise entre 15 et 30 %.

La silicone oxyalkylénée est, de préférence, présente dans la composition à une concentration relative en poids en matière active, comprise préférentiellement entre 0,5 et 5 %, et plus préférentiellement encore entre 1 et 3 %.

La concentration relative en poids en polymère fixant dans la composition est avantageusement comprise entre 0,1 et 20 % en poids, et plus avantageusement encore entre 0,5 et 10 %.

Le milieu cosmétiquement acceptable est de préférence constitué par de l'eau ou un ou plusieurs solvants cosmétiquement acceptables tels que des monoalcools, des polyalcools, des éthers de glycol ou des esters d'acides gras ou des mélanges eau-solvant(s), ces solvants étant de préférence des alcools en C₁-C₄.

Les compositions de l'invention peuvent également contenir en outre au moins un additifs choisi dans le groupe comprenant les corps gras, les agents épaississants, les adoucissants , les agents hydratants, les colorants, les parfums, les conservateurs, les tensioactifs, les protéines et les vitamines.

Ces additifs sont éventuellement présents dans la composition selon l'invention dans des proportions pouvant aller, avantageusement, de 0,001 à 20% en poids par rapport au poids total de la composition. La quantité précise de chaque additif est fonction de sa nature et est déterminée facilement par l'homme de l'art, et dépendra de l'application capillaire choisie.

En particulier, on peut ajouter un coémulsifiant, de préférence non ionique, du type Polysorbate 20, ou encore un électrolyte comme, par exemple, le chlorure de sodium ou le sulfate de magnésium et/ou des agents émollients de type polyols comme la glycérine ou le propylène glycol.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions de l'invention sous forme d'émulsion eau-dans-silicone peuvent être utilisées pour la fabrication de nombreux produits capillaires, comme, par exemple, des produits pour la fixation et/ou le maintien des cheveux, des produits de conditionnement, comme des formulations de brillance ou encore des produits de soin des cheveux.

Ces formulations capillaires selon l'invention seront, de préférence, conditionnées en tube ou en pot.

L'invention pourra être mieux comprise à l'aide des exemples non limitatifs qui suivent et qui constituent des modes de réalisation avantageux des compositions conformes à l'invention.

Toutes les quantités sont exprimées en pourcentages relatifs en poids par rapport au poids total de la composition et m.a. signifie matière active.

Les produits utilisés dans les exemples sont énumérés ci-après :

| | |
|---|---|
| DC 245 Fluid | Silicone non oxyalkylénée commercialisée par Dow Corning |
| DC 200 Fluid | Silicone non oxyalkylénée commercialisée par Dow Corning |
| DC 5225 C | Silicone oxyalkylénée émulsionnante commercialisée par Dow Coming |
| DC 556 Fluid | Silicone non oxyalkylénée commercialisée par Dow Corning |
| DC 2-5185C | Silicone oxyalkylénée émulsionnante commercialisée par Dow Corning |
| Montanox 20 | Polysorbate 20 commercialisé par Seppic |
| Luviquat HM 552 | Polyquaternium 16 commercialisé par Basf |
| Luviskol VA 64 P | Copolymère PVP/VA commercialisé par Basf |
| Amerhold DR25 | Copolymère acrylate commercialisé par Amerchol |

### Exemples:

On réalise les émulsions ci-après conformes à la présente invention.

| **Emulsion coiffante 1** | |
|---|---|
| DC 245 Fluid (Dow Corning) | 7 |
| DC 5225 C (Dow Corning) | 7 |
| DC 200 Fluid (Dow Corning) | 5 |
| NaCI | 1 |
| Montanox 20 (Seppic) | 0.5 |
| Luviquat HM 552 (BASF) | 2 |
| Luviskol VA 64 P (BASF) | 2 |
| Conservateur | qs |
| Eau | qsp |
| | 100 |

| **Emulsion coiffante 2** | |
|---|---|
| DC 245 Fluid (Dow Corning) | 15 |
| DC 5225 C (Dow Corning) | 10 |
| DC 200 Fluid (Dow Corning) | 15 |
| NaCl | 1 |
| Montanox 20 (Seppic) | 0.5 |
| Amerhold DR 25 (Amerchol)) | 16 |
| AMP | 0.35 |
| Conservateur | qs |
| Eau | qsp |
| | 100 |

| **Emulsion de brillance 3** | |
|---|---|
| DC 245 Fluid (Dow Corning) | 7 |
| DC 5225 C (Dow Corning) | 10 |
| Propylène Glycol | 38 |
| NaCI | 1 |
| Luviskol VA 64 P (BASF) | 0.1 |
| Ethanol | 11.2 |
| Conservateur | qs |
| Eau | qsp |
| | 100 |

| **Emulsion de brillance 4** | |
|---|---|
| DC 245 Fluid (Dow Corning) | 10 |
| DC 2-5185C (Dow Corning) | 5 |
| DC 556 Fluid (Dow Coming) | 15 |
| NaCI | 1 |
| Montanox 20 (Seppic) | 1 |
| Aristoflex A (Clariant) | 3.33 |
| AMP | 0.25 |
| Conservateur | qs |
| Eau | qsp |
| | 100 |

| **Emulsion de soin 5** | |
|---|---|
| DC 245 Fluid (Dow Corning) | 5 |
| DC 5225 C (Dow Corning) | 7 |
| DC 1501 Fluid (Dow Corning) | 10 |
| NaCI | 1 |
| Montanox 20 (Seppic) | 0.5 |
| DC 2 1388 Emulsion | 10 |
| Luviskol VA 64 P | 0.1 |
| Conservateur | qs |
| Eau | qsp |
| | 100 |

## Revendications

1. Composition cosmétique capillaire se présentant sous la forme d'émulsion eau-dans-silicone, **caractérisée en ce qu**'elle comprend, dans un milieu cosmétiquement acceptable:
(I) entre 10 % et 30 % en poids d'une silicone non oxyalkylénée choisie(s) parmi les silicones linéaires, cycliques, volatiles ou non, arylées ou non;
(II) au moins une silicone oxyalkylénée émulsionnante dans une proportion de 0.1 à 10 % en poids, et
au moins un polymère fixant anionique, cationique, amphotère ou non ionique.
ladite silicone oxyalkylénée étant choisie parmi les composés de formule générale (I): 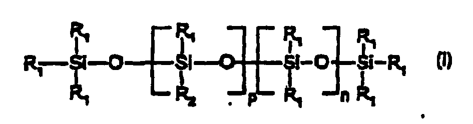 formule dans laquelle :
- R₁, identique ou différent, représente un atome d'hydrogène, un radical alkyle linéaire ou ramifié en C₁-C₃₀ ou un radical phényle,
- R₂, identique ou différent, représente -(CₓH₂ₓ)-(OC₂H₄)ₐ-(OC₃H₆)_{b}-OR₃,
- R₃, identique ou différent, est choisi parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 0 à 1000,
- p varie de 1 à 8,
- a varie de 0 à 50,
- b varie de 0 à 50,
- a + b est supérieur ou égal à 1,
- x varie de 1 à 5,
le poids moléculaire moyen en nombre étant supérieur ou égal à 15.000 et de préférence compris entre 25.000 et 75.000, ayant une viscosité mesurée au moyen du Rhéomat RM 180 à 25°C, avec une lecture après 30 secondes supérieure à 0,5 Pas à un taux de cisaillement de 200 s⁻¹.

2. Composition selon la revendication 1, **caractérisée par le fait que** la silicone non oxyalkylénée est présente à une concentration relative en poids comprise entre 15 et 30 %.

3. Composition selon l'une quelconque des revendications précédentes, **caractérisée par le fait que** la silicone oxyalkylénée est présente dans la composition, à une concentration relative en poids en matière active, comprise entre 0,5 et 5 %, et plus préférentiellement encore entre 1 et 3 %.

4. Composition selon l'une quelconque des revendications précédentes. **caractérisée par le fait que** la concentration relative en poids en polymère fixant est comprise entre 0,1 et 20 % en poids, et de préférence entre 0,5 et 10 %.

5. Composition selon l'une quelconque des revendications précédentes, **caractérisée par le fait qu'**elle comprend en outre un coémulsifiant.

6. Composition selon l'une quelconque des revendications précédentes, **caractérisée par le fait qu'**elle comprend en outre un électrolyte.

7. Composition selon l'une quelconque des revendications précédentes, **caractérisée par le fait que** le polymère fixant est un polymère anionique choisi parmi :
- les polymères comportant des motifs carboxyliques dérivant de monomères mono ou diacides carboxyliques insaturés de formule : 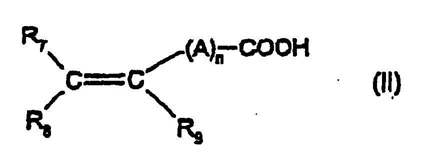 dans laquelle n est un nombre entier de 0 à 10, A désigne un groupement méthylène, éventuellement relié à l'atome de carbone du groupement insaturé
ou au groupement méthylène voisin lorsque n est supérieur à 1 par l'intermédiaire d'un hétématome tel que oxygène ou soufre, R₇ désigne un atome d'hydrogène, un groupement phényle ou benzyle. R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle :
dans la formule précitée, le radical alkyle inférieur désignant un groupement ayant 1 à 4 atomes de carbone;
- les polymères comprenant des motifs dérivant d'acide sulfonique tels que des motife vinylsulfonique, styrènesulfonique. acrylamido alkylsulfonique.

8. Composition selon l'une quelconque des revendications 1 à 6, **caractérisée en ce que** le polymère fixant est un polymère amphotère choisi parmi
(1) les polymères résultant de la copolymérisation d'un monomère dérivé d'un composé vinylique portant un groupement carboxylique;
(2) les polymères comportant des motifs dérivant :
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
(3) les polyamino amides réticulés et alcoylés partiellement ou totalement dérivant de polyaminoamides de formule générale III: 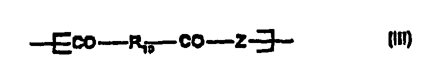 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire;
(4) les polymères comportant des motifs zwittérioniques de formule V: 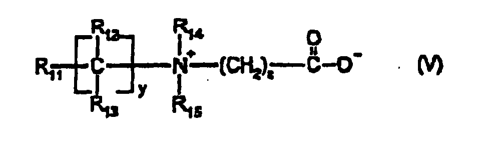 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10;
(5) les polymères dérivés du chitosane comportant des motifs monomères répondant aux formules suivantes : 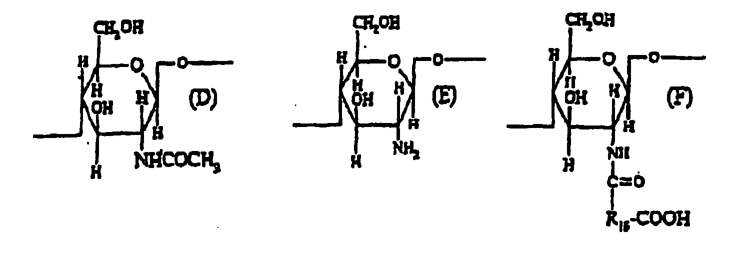 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 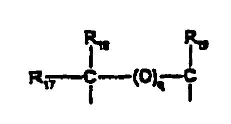 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoyiamine éventuellement interrompus par un ou plusieurs atomes d'azote et/ou éventuellement substitués par un ou plusieurs groupes aminé, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
ou si q=1, R₁₇, R₁₈ et R₁₉ représentent chacun un atome d'hydrogène, ainsi que les sels formés par ces composés avec des bases ou des acides;
(6) Les polymères dérivés de la N-carboxyalkylation du chitosane;
(7) Les polymères répondant à la formule générale (VI) 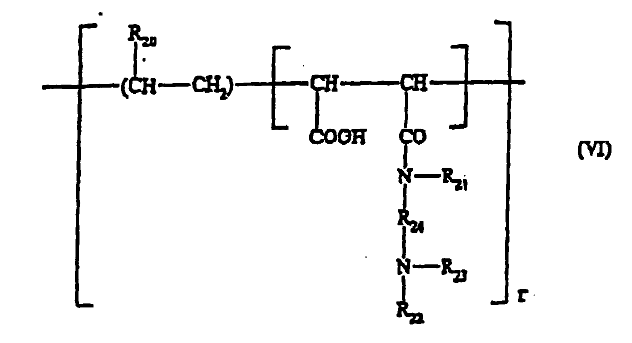 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus,
ainsi que les homologues supérieurs de ces radicaux et contenant jusqu'à 6 atomes de carbone;
(8) Des polymères amphotères du type -D-X-D-X- choisis parmi:
a) les polymères obtenus par action de l'acide chloracétique ou le chloracétate de sodium sur les composés comportant au moins un motif de formule :
-D-X-D-X-D- (VII)
où D désigne un radical 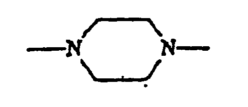 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide. imide, alcool, ester et/ou uréthanne,
b) Les polymères de formule:
-D-X-D-X- (VII')
où D désigne un radical 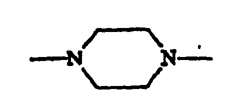 et X désigne le symbole E ou E' et au moins une fois E'; E ayant la signification indiquée ci-dessus et E' est un radical bivalent qui est un radical alkylène à chaîne droite ou ramlfiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride malêlque modifié partiellement par semiamidification avec une N,N-dialkylaminoalkylamine telle que la N,N-diméthylaminopropylamine ou par semiestérification avec une N,N-dialcanolamine.

9. Composition selon l'une quelconque des revendications 1 à 6, **caractérisée par le fait que** le polymère fixant est un polymère non ionique choisi parmi:
- les homopolymères de vinylpyrrolidone ;
- les copolymères de vinylpyrrolidone et d'acétate de vinyle ;
- les polyalkyloxazolines
- les homopolymères d'acétate de vinyle;
- les copolymères d'acétate de vinyle et d'ester acrylique;
- les copolymères d'acètate de vinyle et d'éthylène
- les copolymères d'acétate de vinyle et d'ester maléïque par exemple de maléate de dibutyle;
- les copolymères de polyéthylène et d'anhydride maléïque ;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyles;
- les copolymères d'acrylonitrile et d'un monomère non ionique choisi par exemple parmi le butadiène et les (méth)acrylates d'alkyle ;
- les polyuréthannes;
- les copolymères d'acrylate d'alkyle et d'uréthanne;
- les polyamides;
- les gommes de guar non ioniques chimiquement modifiées ou non modifiées.

10. Composition selon la revendication 1 à 6, **caractérisée par le fait que** le polymère fixant est un polymère cationique choisi parmi :
- le copolymère d'acrylamide et de diméthylaminoéthyl méthacrylate quaternisé au sulfate de diméthyle,
- les copolymères d'acrylamide et de chlorure de méthacryloyloxyéthyltriméthylammonium,
- le copolymère d'acrylamido et de méthosulfate de méthacryloyloxyéthyltriméthylammonium,
- les copolymères vinylpyrrolidone I acrylate ou méthacrylate de dialkylaminoalkyle quaternisés ou non,
- les terpolymère méthacrylate de diméthyl amino éthyle/ vinylcaprolactame/vinylpyrrolidone.
- le copolymère vinylpyrrolidone / méthacrylamide de diméthylaminopropyle quaternisé,
- les polysaccharides quaternisés, tels que les gommes de guar contenant des groupements cationiques trialkylammonium,
- les copolymères quaternaires de vinylpyrrolidone et de vinylimidazole,
- les chitosanes ou leurs sels,
- les dérivés de cellulose cationiques.

11. Composition selon l'une quelconque des revendications 1 à 6, **caractérisée par le fait que** le polymère fixant est un povuréthanne fonctionnalisé ou non, siliconé ou non.

12. Composition selon l'une quelconque des revendications 1 à 6, **caractérisée par le fait que** le polymère fixant est un polymère de type siliconé greffé comprenant une portion polysiloxane et une portion constituée d'une chaîne organique non-siliconée, l'une des deux portions constituant la chaîne principale du polymère l'autre étant greffée sur la dite chaîne principale.

13. Composition selon l'une quelconque des revendications précédentes, **caractérisée par le fait qu'**elle contient en outre des additifs cosmétiques choisis dans le groupe comprenant les corps gras, les agents épaississants, les adoucissants, les agents hydratants, les colorants, les parfums, les conservateurs, les tensioactifs, les protéines et les vitamines.

14. Composition selon l'une quelconque des revendications précédentes, **caractérisée par le fait qu'**elle est conditionnée sous forme de tube ou de pot.

15. Procédé cosmétique capillaire, **caractérisé par le fait qu'**on met en oeuvre une composition conforme à l'une quelconque des revendications 1 à 14.

16. Procédé selon la revendication 15, où la composition est une composition de fixation et/ou de maintien des cheveux.

17. Utilisation d'une composition selon l'une quelconque des revendications 1 à 14, pour la fabrication d'un produit cosmétique capillaire.

18. Utilisation selon la revendication 17 où le produit capillaire est un produit pour le maintien et/ou la fixation des cheveux.

19. Utilisation selon la revendication 17, où le produit capillaire est un produit de conditionnement des cheveux, comme un produit de brillance.

20. Utilisation selon la revendication 17, où le produit capillaire est un produit de soin des cheveux.

21. Produit capillaire pour le maintien et/ou la fixation des cheveux, **caractérisé par le fait qu'**il comprend une composition selon l'une quelconque des revendications 1 à 14.

## Claims

1. Hair cosmetic composition which is provided in the form of a water-in-silicone emulsion, **characterized in that** it comprises, in a cosmetically acceptable medium:
(i) between 10% and 30% by weight of a non-oxyalkylenated silicone chosen from linear or cyclic, volatile or non-volatile and arylated or non-arylated silicones;
(ii) at least one emulsifying oxyalkylenated silicone in a proportion of 0.1 to 10% by weight; and
(iii) at least one anionic, cationic, amphoteric or non-ionic fixing polymer;
the said oxyalkylenated silicone being chosen from the compounds of general formula (I): 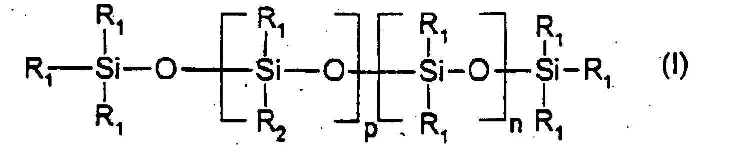 in which formula:
- R₁, which is identical or different, represents a hydrogen atom, a linear or branched C₁-C₃₀ alkyl radical or a phenyl radical,
- R₂, which is identical or different, represents
- (CₓH₂ₓ) - (OC₂H₄) a- (OC₃H₆) _{b}-OR₃,
- R₃, which is identical or different, is chosen from a hydrogen atom, a linear or branched alkyl radical having from 1 to 12 carbon atoms or a linear or branched acyl radical having from 2 to 12 carbon atoms,
- n varies from 0 to 1000,
- p varies from 1 to 8,
- a varies from 0 to 50,
- b varies from 0 to 50,
- a + b is greater than or equal to 1,
- x varies from 1 to 5,
the number-average molecular weight being greater than or equal to 15,000 and preferably between 25,000 and 75,000, having a viscosity, measured by means of a Rheomat RM 180 at 25°C with reading after 30 seconds, of greater than 0.5 Pa.s, at a shear rate of 2.00 s⁻¹.

2. Composition according to Claim 1, **characterized in that** the non-oxyalkylenated silicone is present at a relative concentration by weight of between 15 and 30%. .

3. Composition according to any one of the preceding claims, **characterized in that** the oxyalkylenated silicone is present in the composition at a relative concentration by weight of active material of between 0.5 and 5% and more preferably still between 1 and 3%.

4. Composition according to any one of the preceding claims, **characterized in that** the relative concentration by weight of fixing polymer is between 0.1 and 20% by weight and preferably between 0.5 and 10%.

5. Composition according to any one of the preceding claims, **characterized in that** it additionally comprises a coemulsifier.

6. Composition according to any one of the preceding claims, **characterized in that** it additionally comprises an electrolyte.

7. Composition according to any one of the preceding claims, **characterized in that** the fixing polymer is an anionic polymer chosen from:
- polymers comprising carboxyl units deriving from unsaturated carboxylic mono- or diacid monomers of formula: 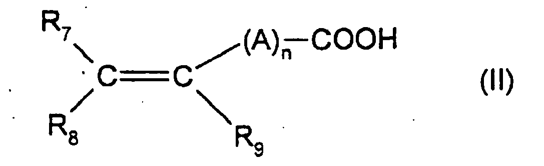 in which n is an integer from 0 to 10, A denotes a methylene group, optionally bonded to the carbon atom of the unsaturated group or to the neighbouring methylene group when n is greater than 1 via a heteroatom, such as oxygen or sulphur, R₇ denotes a hydrogen atom or a phenyl or benzyl group, R₈ denotes a hydrogen atom or a lower alkyl or carboxyl group, and R₉ denotes a hydrogen atom, a lower alkyl group or a -CH₂-COOH, phenyl or benzyl group;
the lower alkyl radical denoting, in the abovementioned formula, a group having 1 to 4 carbon atoms;
- polymers comprising units deriving from sulphonic acid, such as vinylsulphonic, styrenesulphonic or acrylamidoalkylsulphonic units.

8. Composition according to any one of Claims 1 to 6, **characterized in that** the fixing polymer is an amphoteric polymer chosen from
(1) polymers resulting from copolymerization of a monomer derived from a vinyl compound carrying a carboxyl group;
(2) polymers comprising units deriving:
a) from at least one monomer chosen from acrylamides or methacrylamides substituted on the nitrogen by an alkyl radical,
b) from at least one acidic comonomer comprising one or more reactive carboxyl groups, and
c) from at least one basic comonomer, such as esters comprising primary, secondary, tertiary and quaternary amine substituents of acrylic and methacrylic acids and the quaternization product of dimethylaminoethyl methacrylate with dimethyl or diethyl sulphate,
(3) partially or completely alkylated and crosslinked polyaminoamides deriving from polyaminoamides of general formula III: 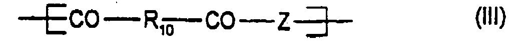 in which R₁₀ represents a divalent radical derived from a saturated dicarboxylic acid, from an aliphatic mono- or dicarboxylic acid comprising an ethylenic double bond, from an ester of a lower alkanol having 1 to 6 carbon atoms of these acids, or from a radical deriving from the addition of any one of the said acids with a bisprimary or bissecondary amine, and Z denotes a radical of a bisprimary, mono- or bissecondary polyalkylenepolyamine;
(4) polymers comprising zwitterionic units of formula V: 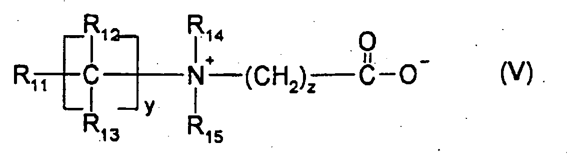 in which R₁₁ denotes a polymerizable unsaturated group, such as an acrylate, methacrylate, acrylamide or methacrylamide group, y and z represent an integer from 1 to 3, R₁₂ and R₁₃ represent a hydrogen atom or methyl, ethyl or propyl, and R₁₄ and R₁₅ represent a hydrogen atom or an alkyl radical such that the sum of the carbon atoms in R₁₄ and R₁₅ does not exceed 10;
(5) polymers derived from chitosan comprising monomer units corresponding to the following formulae: 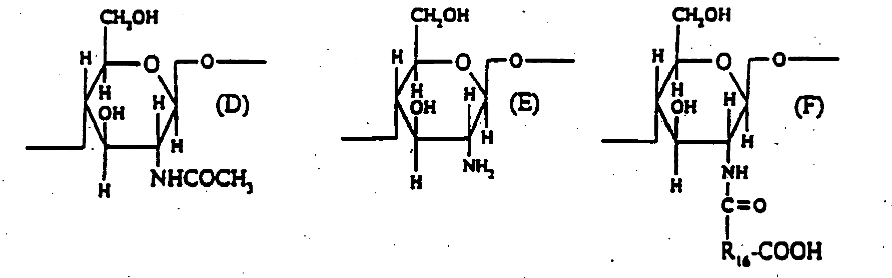 the unit D being present in proportions of between 0 and 30%; the unit E in proportions of between 5 and 50% and the unit F in proportions of between 30 and 90%, it being understood that, in this unit F, R₁₆ represents a radical of formula: 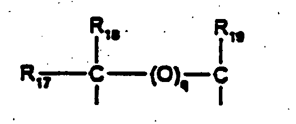 in which, if q=0, R₁₇, R₁₈ and R_{19.} which art identical or different, each represent a hydrogen atom, a methyl, hydroxyl, acetoxy or amino residue, a monoalkylamine residue or a dialkylamine residue, optionally interrupted by one or more nitrogen atoms and/or optionally substituted by one or more amine, hydroxyl, carboxy, alkylthio or sulpho groups, or an alkylthio residue in which the alkyl group carries an amino residue, at least one of the R₁₇, R₁₈ and R₁₉ radicals being, in this case, a hydrogen atom;
or; if q=1, R₁₇, R₁₈ and R₁₉ each represent a hydrogen atom, and the salts formed by these compounds with bases or acids;
(6) Polymers derived from the N-carboxyalkylation of chitosan;
(7) Polymers corresponding to the general formula (VI): 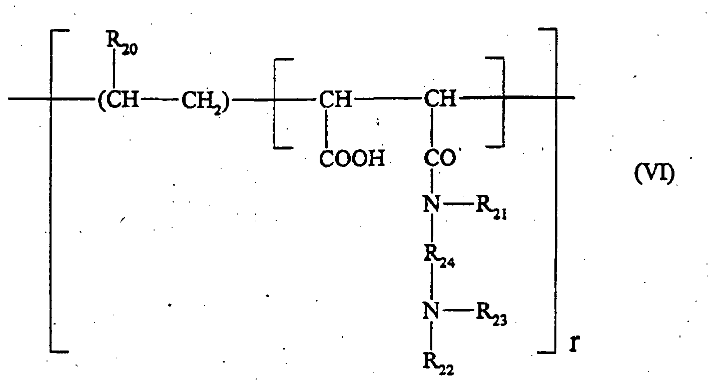 in which R₂₀ represents a hydrogen atom or a CH₃O, CH₃CH₂O or phenyl radical, R₂₁ denotes hydrogen or a lower alkyl radical such as methyl or ethyl, R₂₂ denotes hydrogen or a lower alkyl radical such as methyl or ethyl and R₂₃ denotes a lower alkyl radical such as methyl or ethyl or a radical corresponding to the formula: -R₂₄-N(R₂₂)₂, R₂₄ representing a -CH₂-CH₂-, -CH₂-CH₂-CH₂- or -CH₂-CH(CH₃)- group and R₂₂ having the meanings mentioned above, and the higher homologues of these radicals comprising up to 6 carbon atoms;
(8) Amphoteric polymers of the -D-X-D-X- type chosen from:
a) polymers obtained by reaction of chloroacetic acid or sodium chloroacetate with compounds comprising at least one unit of formula:
-D-X-D-X-D- (VII)
where D denotes a radical 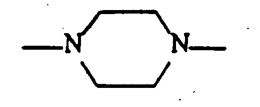 and X denotes the symbol E or E', E or E', which are identical or different, denote a bivalent radical which is a straight- or branched-chain alkylene radical comprising up to 7 carbon atoms in the main chain, which chain is unsubstituted or substituted by hydroxyl groups, and which can additionally comprise oxygen, nitrogen or sulphur atoms or 1 to 3 aromatic and/or heterocyclic rings; the oxygen, nitrogen and sulphur atoms being present in the form of ether, thioether, sulphoxide, sulphone, sulphonium, alkylamine or alkenylamine groups or hydroxyl, benzylamine, amine oxide, quaternary ammonium, amide, imide, alcohol, ester and/or urethane groups,
b) Polymers of formula:
-D-X-D-X- (VII')
where D denotes a radical 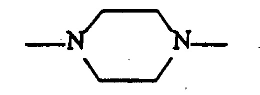 and X denotes the symbol E or E' and E' at least once; E having the meaning indicated above and E' is a bivalent radical which is a straight- or branched-chain alkylene radical having up to 7 carbon atoms in the main chain and which is substituted or unsubstituted by one or more hydroxyl radicals and which comprises one or more nitrogen atoms, the nitrogen atom being substituted by an alkyl chain optionally interrupted by an oxygen atom and necessarily comprising one or more carboxyl functional groups or one or more hydroxyl functional groups and betainized by reaction with chloroacetic acid or sodium chloroacetate;
(9) (C₁-C₅)alkyl vinyl ether/maleic anhydride copolymers which are partially modified by semiamidation with an N,N-dialkylaminoalkylamine, such as N,N-dimethylaminopropylamine, or by semiesterification with an N,N-dialkanolamine.

9. Composition according to any one of Claims 1 to 6, **characterized in that** the fixing polymer is a non-ionic polymer chosen from:
- vinylpyrrolidone homopolymers;
- copolymers of vinylpyrrolidone and of vinyl acetate;
- polyalkyloxazolines;
- vinyl acetate homopolymers;
- copolymers of vinyl acetate and of acrylic ester;
- copolymers of vinyl acetate and of ethylene;
- copolymers of vinyl acetate and of maleic ester, for example dibutyl maleate;
- copolymers of polyethylene and of maleic anhydride;
- alkyl acrylate homopolymers and alkyl methacrylate homopolymers;
- acrylic ester copolymers, such as, for example, copolymers of alkyl acrylates and of alkyl methacrylates;
- copolymers of acrylonitrile and of a non-ionic monomer chosen, for example, from butadiene and alkyl (meth)acrylates;
- polyurethanes;
- copolymers of alkyl acrylate and of urethane;
- polyamides;
- chemically modified or unmodified non-ionic guar gums.

10. Composition according to Claim 1 to 6, **characterized in that** the fixing polymer is a cationic polymer chosen from:
- the copolymer of acrylamide and of dimethylaminoethyl methacrylate quaternized with dimethyl sulphate,
- copolymers of acrylamide and of methacryloyloxy-ethyltrimethylammonium chloride,
- the copolymer of acrylamide and of methacryloyloxy-ethyltrimethylammonium methyl sulphate,
- optionally quaternized vinylpyrrolidone/dialkylaminoalkyl acrylate or methacrylate copolymers,
- dimethylaminoethyl methacrylate/vinylcaprolactam/vinylpyrrolidone terpolymers,
- the quaternized dimethylaminopropyl methacrylamide/vinylpyrrolidone copolymer, '
- quaternized polysaccharides, such as guar gums comprising cationic trialkylammonium groups,
- quaternary copolymers of vinylpyrrolidone and of vinylimidazole,
- chitosans or their salts,
- cationic cellulose derivatives.

11. Composition according to any one of Claims 1 to 6, **characterized in that** the fixing polymer is a functionalized or non-functionalized and silicone-comprising or non-silicone-comprising polyurethane.

12. Composition according to any one of Claims 1 to 6, **characterized in that** the fixing polymer is a polymer of grafted silicone type comprising a polysiloxane portion and a portion composed of a non-silicone organic chain, one of the two portions constituting the main chain of the polymer and the other being grafted onto the said main chain.

13. Composition according to any one of the preceding claims, **characterized in that** it additionally comprises cosmetic additives chosen from the group consisting of fatty substances, thickening agents, softeners, moisturizing agents, colorants, fragrances, preservatives, surfactants, proteins and vitamins.

14. Composition according to any one of the preceding claims, **characterized in that** it is packaged in the form of tubes or pots.

15. Hair cosmetic process, **characterized in that** use is made of a composition in accordance with any one of Claims 1 to 14.

16. Process according to Claim 15, where the composition is a composition , for fixing and/or retaining the form of the hair.

17. Use of a composition according to any one of Claims 1 to 14 in the manufacture of a hair cosmetic product.

18. Use according to Claim 17, where the hair product is a product for retaining the form of and/or fixing the hair.

19. Use according to Claim 17, where the hair product is a conditioner for the hair, such as a sheen product.

20. Use according to Claim 17, where the hair product is a hair care product.

21. Hair product for retaining the form of and/or fixing the hair, **characterized in that** it comprises a composition according to any one of Claims 1 to 14.

## Patentansprüche

1. Kosmetische Zusammensetzung für das Haar, die in Form einer Wasser-in-Silicon-Emulsion vorliegt, **dadurch gekennzeichnet, dass** sie in einem kosmetisch akzeptablen Medium enthält:
(i) 10 bis 30 Gew.-% nicht oxyalkyliertes Silicon, das (die) unter den geradkettigen, cyclischen, flüchtigen oder nicht flüchtigen, arylierten oder nicht arylierten Siliconen ausgewählt ist (sind),
(ii) mindestens ein emulgierendes oxyalkyliertes Silicon in einem Mengenanteil von 0,1 bis 10 Gew.-%, und
(iii) mindestens ein anionisches, kationisches, amphoteres oder nichtionisches fixierendes Polymer.
wobei das oxyalkylierte Silicon unter den Verbindungen der allgemeinen Formel (I) ausgewählt ist: 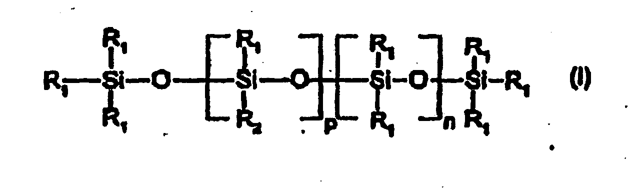 wobei in der Formel:
- die Gruppen R₁, die identisch oder voneinander verschieden sind, ein Wasserstoffatom, eine geradkettige oder verzweigte C₁₋₃₀-Alkylgruppe oder eine Phenylgruppe bedeuten,
- die Gruppen R₂, die identisch oder voneinander verschieden sind, -(CₓH₂ₓ)-(OC₂H₄)ₐ-(OC₃H₆)_{b}-OR₃ bedeuten,
- die Gruppen R₃, die identisch oder voneinander verschieden sind, unter einem Wasserstoffatom, einer geradkettigen oder verzweigten Alkylgruppe mit 1 bis 12 Kohlenstoffatomen und einer geradkettigen oder verzweigten Acylgruppe mit 2 bis 12 Kohlenstoffatomen ausgewählt sind,
- n im Bereich von 0 bis 1 000 liegt,
- p im Bereich von 1 bis 8 liegt,
- a im Bereich von 0 bis 50 liegt,
- b im Bereich von 0 bis 50 liegt,
- a + b größer oder gleich 1 ist, und
- x im Bereich von 1 bis 5 liegt,
wobei das Zahlenmittel der Molmasse mindestens 15 000 beträgt und vorzugsweise im Bereich von 25 000 bis 75 000 liegt,
wobei sie eine mit einem Viskosimeter RHEOMAT RM 180 bei 25 °C bestimmte Viskosität über 0,5 Pa.s aufweist, wobei nach 30 s abgelesen wird und die Messung bei einer Schergeschwindigkeit von 200 s⁻¹ erfolgt.

2. Zusammensetzung nach Anspruch 1, **dadurch gekennzeichnet, dass** das nicht oxyalkylierte Silicon in der Zusammensetzung in einer auf das Gewicht bezogenen relativen Konzentration von 15 bis 30 % vorliegt.

3. Zusammensetzung nach einem der vorhergehenden Ansprüche, **dadurch gekennzeichnet, dass** das oxyalkylierte Silicon in der Zusammensetzung in einer auf das Gewicht bezogenen relativen Konzentration von 0,5 bis 5 % und besonders bevorzugt 1 bis 3 % enthalten ist.

4. Zusammensetzung nach einem der vorhergehenden Ansprüche, **dadurch gekennzeichnet, dass** die auf das Gewicht bezogene, relative Konzentration des fixierenden Polymers im Bereich von 0,1 bis 20 Gew.-% und vorzugsweise 0,5 bis 10 Gew.-% liegt.

5. Zusammensetzung nach einem der vorhergehenden Ansprüche, **dadurch gekennzeichnet, dass** sie ferner einen Coemulgator enthält.

6. Zusammensetzung nach einem der vorhergehenden Ansprüche, **dadurch gekennzeichnet, dass** sie ferner einen Elektrolyten enthält.

7. Zusammensetzung nach einem der vorhergehenden Ansprüche, **dadurch gekennzeichnet, dass** das fixierende Polymer ein anionisches Polymer ist, das ausgewählt ist unter:
- den Polymeren, die Carboxyeinheiten aufweisen, die von ungesättigten Mono- oder Dicarbonsäuremonomeren der Formel: 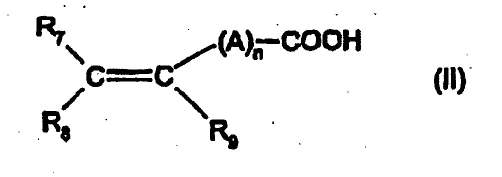 abgeleitet sind, wobei n 0 oder eine ganze Zahl von 1 bis 10 bedeutet, A eine Methylengruppe ist, die gegebenenfalls an das Kohlenstoffatom der ungesättigten Gruppe oder, wenn n größer 1 ist, an die benachbarte Methylengruppe über ein Heteroatom, wie Sauerstoff oder Schwefel, gebunden ist, R₇ Wasserstoffs Phenyl oder Benzyl bedeutet, R₈ Wasserstoff, eine niedere Alkylgruppe oder Carboxy ist und R⁹ Wasserstoff, eine niedere AlkylGruppe, -CH₂-COOH, Phenyl oder Benzyl bedeutet, wobei in der Formel unter einer niederen Alkylgruppe eine Gruppe mit 1 bis 4 Kohlenstoffatomen verstanden wird; und
- den Polymeren, die Einheiten enthalten, die von Sulfonsäuren abgeleitet sind, wie Vinylsulfonsäureeinheiten, Styrolsulfonsäureeinheiten und Acrylamidoalkylsulfonsäureeinheiten.

8. Zusammensetzung nach einem der Ansprüche 1 bis 6, **dadurch gekennzeichnet, dass** das fixierende Polymer ein amphoteres Polymer ist, das ausgewählt ist unter:
(1) den Polymeren, die bei der Copolymerisation eines von einer Vinylverbindung abgeleiteten Monomers mit Carboxygruppe entstehen;
(2) den Polymeren, die Einheiten aufweisen, welche abgeleitet sind von:
a) mindestens einem Monomer, das unter den Acrylamiden oder Methacrylamiden ausgewählt ist, die am Stickstoffatom mit einer Alkylgruppe substituiert sind,
b) mindestens einem Säurecomonomer, das eine oder mehrere reaktive Carboxygruppen aufsweist, und
c) mindestens einem basischen Comonomer, beispielsweise Acrylsäureestern und Methacrylsäureestern mit primären, sekundären, tertiären und quartären Aminosubstituenten, und dem Quaternisierungsprodukt von Dimethylaminoethylmethacrylat und Dimethylsulfat oder Diethylsulfat;
(3) den Polyaminoamiden, die ganz oder teilweise alkyliert und vernetzt sind und von Polyaminoamiden der allgemeinen Formel (III) abgeleitet sind: 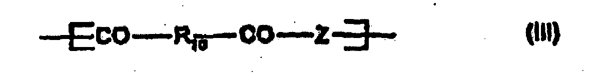 worin R₁₀ eine zweiwertige Gruppe bedeutet, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe, die aus der Umsetzung einer dieser Säuren mit einem bis-primären oder bis-sekundären Amin stammt, abgeleitet ist; und Z eine bis-primäre, mono oder bis-sekundäre-Polyalkylen-Polyamin-Gruppe bedeutet;
(4) den Polymeren mit zwitterionischen Einheiten der folgenden Formel (V): 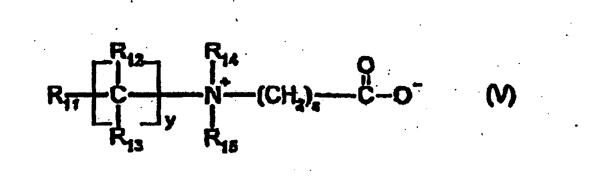 worin R₁₁ eine ungesättigte, polymerisierbare Gruppe, wie Acrylat, Methacrylat, Acrylamid oder Methacrylamid, y und z eine ganze Zahl im Bereich von 1 bis 3, R₁₂ und R₁₃ Wasserstoff, Methyl, Ethyl oder Propyl, R₁₄ und R₁₅ Wasserstoff oder Alkyl bedeuten, mit der Maßgabe, dass die Summe der Kohlenstoffatome in den Gruppen R₁₄ und R₁₅ 10 nicht übersteigt;
(5) den von Chitosan abgeleiteten Polymeren, die Monomereinheiten der folgenden Formeln enthalten: 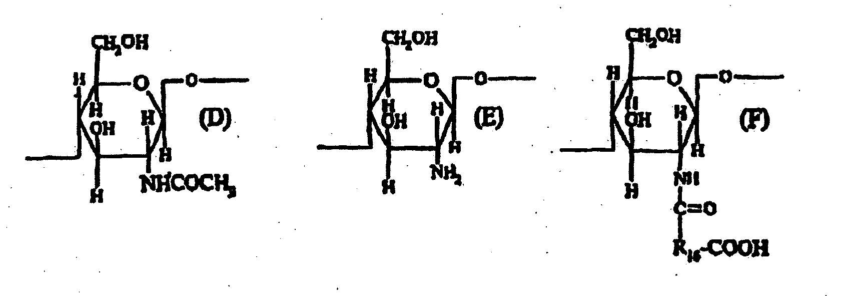 wobei die Einheit D in Anteilen von 0 bis 30 %, die Einheit E in Anteilen von 5 bis 50 % und die Einheit F in Anteilen von 30 bis 90 % vorliegt, mit der Maßgabe, dass in der Einheit F die Gruppe R₁₆ die folgende Gruppe bedeutet: 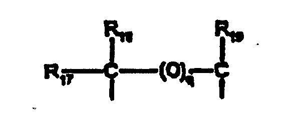 worin, wenn q = 0 ist, die Gruppen R₁₇, R₁₈ und R₁₉, die identisch oder voneinander verschieden sind, Wasserstoff, Methyl, Hydroxy, Acetoxy, Amino, eine Monoalkylamin- oder Dialkylaminogruppe, die gegebenenfalls durch ein oder mehrere Stickstoffatome unterbrochen und/oder gegebenenfalls mit einer oder mehreren Amino- Hydroxy-, Carboxy-, Alkylthio- oder Sulfonsäuregruppen substituiert sind, oder eine Alkylthiogruppe, deren Alkylgruppe eine Aminogruppe trägt, bedeuten, wobei in diesem Fall mindestens eine der Gruppen R₁₇, R₁₈ und R₁₉ Wasserstoff bedeutet;
oder, wenn q = 1 ist, die Gruppen R₁₇, R₁₈ und R₁₉ Wasserstoff bedeuten,
sowie den Salzen, die diese Verbindungen mit Basen oder Säuren bilden;
(6) den Polymeren, die von N-carboxyalkyliertem Chitosan abgeleitet sind;
(7) den Polymeren, die der allgemeinen Formel (VI) entsprechen: 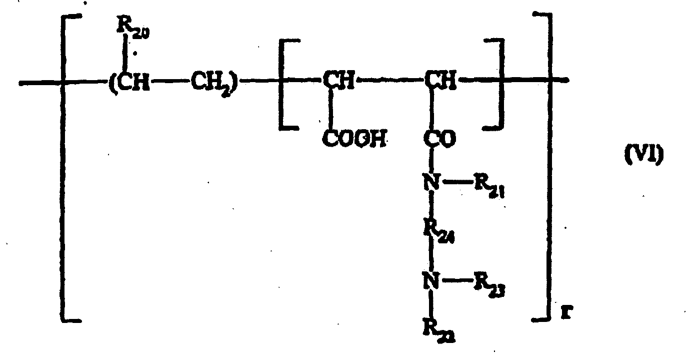 worin bedeuten: R₂₀ Wasserstoff, CH₃O CH₃CH₂O oder Phenyl, R₂₁ Wasserstoff oder eine niedere Alkylgruppe, wie Methyl und Ethyl, R₂₂ Wasserstoff oder eine niedere Alkylgruppe, wie Methyl und Ethyl, und R₂₃ eine niedere Alkylgruppe, wie Methyl und Ethyl, oder eine Gruppe der Formel R₂₄-N(R₂₂)₂, wobei R₂₄-CH₂-CH₂-, -CH₂-CH₂-CH₂- oder -CH₂-CH(CH₃)- bedeutet und R₂₂ die oben angegebenen Bedeutungen aufweist, sowie die höheren Homologen dieser Gruppen, die bis zu 6 Kohlenstoffatome enthalten;
(8) den amphoteren Polymeren vom Typ -D-X-D-X-, die ausgewählt sind unter:
a) den Polymeren, die durch Einwirkung von Chloressigsäure oder Natriumchloracetat auf Verbindungen hergestellt sind, welche mindestens eine Einheit der folgenden Formel aufweisen:
-D-X-D-X-D- (VII)
wobei D die Gruppe 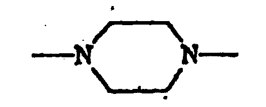 und X E oder E' bedeutet, wobei E und E', die identisch oder voneinander verschieden sind, eine zweiwertige Gruppe bedeuten, die eine geradkettige oder verzweigte Alkylengruppe mit bis zu 7 Kohlenstoffatomen in der Hauptkette ist, die unsubstituiert vorliegt oder mit Hydroxy substituiert ist und ferner Sauerstoff-, Stickstoff- oder Schwefelatome und 1 bis 3 aromatische und/oder heterocyclische Ringe enthalten kann, wobei die Sauerstoff-, Stickstoff- und Schwefelatome in der Form folgender Gruppen vorliegen: Ether, Thioether, Sulfoxid, Sulfon, Sulfonium, Alkylamin, Alkenylamin, Hydroxy, Benzylamin, Aminoxid, quartäres Ammonium, Amid, Imid, Alkohol, Ester und/oder Urethan;
b) den Polymeren der Formel
-D-X-D-X- (VII')
wobei D die Gruppe 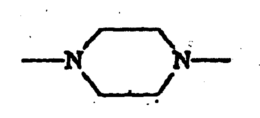 und X E oder E' und mindestens einmal E' bedeutet, wobei E die oben angegebene Bedeutung aufweist und E' eine zweiwertige Gruppe bedeutet, die eine geradkettige oder verzweigte Alkylengruppe mit bis zu 7 Kohlenstoffatomen in der Hauptkette ist, die unsubstituiert vorliegt oder ein- oder mehrfach mit Hydroxy substituiert ist und ein oder mehrere Stickstoffatome aufweist, wobei das Stickstoffatom mit einer Alkylgruppe substituiert ist, die gegebenenfalls durch Sauerstoff unterbrochen ist, zwingend eine oder mehrere Carboxygruppen oder eine oder mehrere Hydroxygruppen enthält und durch Umsetzung mit Chloressigsäure oder Natriumchloracetat in das Betain überführt ist;
(9) den Copolymeren C₁₋₅-Alkylvinylether/Maleinsäureanhydrid, das partiell durch Semiamidierung mit einem N,N-Dialkylaminoalkylamin, wie N,N-Dimethylaminopropylamin, oder Semiveresterung mit einem N,N-Dialkanolamin modifiziert wurde.

9. Zusammensetzung nach einem der Ansprüche 1 bis 6, **dadurch gekennzeichnet, dass** das fixierende Polymer ein nichtionisches Polymer ist, das ausgewählt ist unter:
- den Homopolymeren von Vinylpyrrolidon;
- den Copolymeren von Vinylpyrrolidon und Vinylacetat;
- den Polyalkyloxazolinen;
- den Homopolymeren von Vinylacetat;
- den Copolymeren von Vinylacetat und Acrylestern;
- den Copolymeren von Vinylacetat und Ethylen;
- den Copolymeren von Vinylacetat und Maleinsäureestern, wie Dibutylmaleat;
- den Copolymeren von Polyethylen und Maleinsäureanhydrid;
- den Homopolymeren von Alkylacrylaten und den Homopolymeren von Alkylmethacrylaten;
- den Acrylester-Copolymeren, wie beispielsweise den Copolymeren von Alkylacrylaten und Alkylmethacrylaten;
- den Copolymeren von Acrylnitril und einem nichtionischen Monomer, das beispielsweise unter Butadien und den Alkyl(meth)acrylaten ausgewählt ist;
- den Polyurethanen;
- den Copolymeren von Alkylacrylat und Urethanen;
- den Polyamiden;
- den nichtionischen Guargummen, die chemisch modifiziert oder nicht chemisch modifiziert wurden.

10. Zusammensetzung nach einem der Ansprüche 1 bis 6, **dadurch gekennzeichnet, dass** das fixierende Polymer ein kationisches Polymer ist, das ausgewählt ist unter:
- den Copolymeren von Acrylamid und mit Dimethylsulfat quaternisiertem Dimethylaminoethylmethacrylat,
- den Copolymeren von Acrylamid und Methacryloyloxyethyltrimethylammoniumchlorid,
- dem Copolymer von Acrylamid und Methacryloyloxyethyltrimethylammoniummethosulfat,
- den Copolymeren von Vinylpyrrolidon/Dialkylaminoalkylacrylat oder -methacrylat, die gegebenenfalls quaternisiert sind, .
- den Dimethylaminoethylmethacrylat/Vinylcaprolactam/Vinylpyrrolidon-Terpolymeren,
- dem Copolymer von Vinylpyrrolidon und quaternisiertem Dimethylaminopropylmethacrylamid,
- den quaternisierten Polysacchariden, beispielsweise Guargummen, die kationische Trialkylammoniumgruppen tragen,
- den quartären Copolymeren von Vinylpyrrolidon und Vinylimidazol,
- den Chitosanen oder ihren Salzen,
- den kationischen Cellulosederivaten.

11. Zusammensetzung nach einem der Ansprüche 1 bis 6, **dadurch gekennzeichnet, dass** das fixierende Polymer ein gegebenenfalls funktionalisiertes, gegebenenfalls siliconhaltiges Polyurethan ist.

12. Zusammensetzung nach einem der Ansprüche 1 bis 6, **dadurch gekennzeichnet, dass** das fixierende Polymer ein Polymer vom Typ der gepfropften Siliconpolymere ist, die einen Polysiloxanbereich und einen Bereich enthalten, der aus einer nicht siliconhaltigen organischen Kette besteht, wobei einer der beiden Bereiche die Hauptkette des Polymers bildet und der andere auf die Hauptkette gepfropft ist.

13. Zusammensetzung nach einem der vorhergehenden Ansprüche, **dadurch gekennzeichnet, dass** sie ferner kosmetische Zusatzstoffe enthält, die unter den Fettsubstanzen, Verdickungsmitteln, beruhigenden Stoffen, Hydratisierungsmitteln, Farbmitteln, Parfums, Konservierungsmitteln, grenzflächenaktiven Stoffen, Proteinen und Vitaminen ausgewählt sind.

14. Zusammensetzung nach einem der vorhergehenden Ansprüche, **dadurch gekennzeichnet, dass** sie in Tuben oder Tiegeln konfektioniert ist.

15. Kosmetisches Verfahren für das Haar, **dadurch gekennzeichnet, dass** eine Zusammensetzung nach einem der Ansprüche 1 bis 14 verwendet wird.

16. Verfahren nach Anspruch 15, wobei es sich um eine Zusammensetzung zum Fixieren und/oder für den Halt der Haare handelt.

17. Verwendung einer Zusammensetzung nach einem der Ansprüche 1 bis 14 zur Herstellung eines kosmetischen Produktes für das Haar.

18. Verwendung nach Anspruch 17, wobei es sich um ein Produkt zum Fixieren und/oder für den Halt der Haare handelt.

19. Verwendung nach Anspruch 17, wobei es sich um ein Produkt zum Konditionieren der Haare, beispielsweise ein Produkt für den Glanz handelt.

20. Verwendung nach Anspruch 17, wobei es sich um ein Produkt zur Pflege der Haare handelt.

21. Produkt zum Fixieren und/oder für den Halt der Haare, **dadurch gekennzeichnet, dass** es eine Zusammensetzung nach einem der Ansprüche 1 bis 14 enthält.
